# EUROPÄISCHE PATENTSCHRIFT

(11) **EP 1 881 971 B1**
(45) Veröffentlichungstag und Bekanntmachung des Hinweises auf die Patenterteilung: **11.07.2012**
(21) Anmeldenummer: 06742786.4
(22) Anmeldetag: 04.05.2006
(51) Int. Cl.: C07D 317/40

(54) **HOCHREINES VINYLENCARBONAT SOWIE EIN VERFAHREN ZUR REINIGUNG VON VINYLENCARBONAT**
HIGH-PURITY VINYLENE CARBONATE AND A METHOD OF PURIFYING VINYLENE CARBONATE
CARBONATE DE VINYLENE DE GRANDE PURETE ET PROCEDE DE PURIFICATION DE CARBONATE DE VINYLENE

(30) Priorität: 12.05.2005 DE 102005021967
(43) Veröffentlichungstag der Anmeldung: 30.01.2008
(73) Patentinhaber: Saltigo GmbH, 40764 Langenfeld (DE)
(72) Erfinder: LANGER, Reinhard, 47918 Tönisvorst (DE); WAGNER, Paul, 40597 Düsseldorf (DE); GRZINIA, Heinrich, 41812 Erkelenz (DE)
(74) Vertreter: Pettrich, Klaus-Günter
(86) Internationale Anmeldenummer: PCT/EP2006/004153
(87) Internationale Veröffentlichungsnummer: WO 2006/119907

(56) Entgegenhaltungen:
- US-A1- 2002 107 407
- PATENT ABSTRACTS OF JAPAN Bd. 2003, Nr. 03, 5. Mai 2003 (2003-05-05) & JP 2002 322171 A (MITSUBISHI CHEMICALS CORP), 8. November 2002 (2002-11-08)

## Beschreibung

Die vorliegende Erfindung begriff ein Verfahren zur Reinigung von Vinylencarbonat. Vinylencarbonat (VC) ist ein wichtiges Zwischenprodukt für die Herstellung von Chemikalien, Pharmaprodukten, Pflanzenschutzmitteln und im Besonderen für Polymere, Lacke und Batterieelektrolyte.

Vinylencarbonat wird nach bekannter Methodik durch Abspalten von Chlorwasserstoff aus Chlorethylenglykolcarbonat mittels tertiärer Amine, insbesondere Triethylamin hergestellt.

Das Chlorethylenglykolcarbonat gewinnt man durch radikalische Chlorierung von Ethylenglykolcarbonat mittels Chlor oder Sulfurylchlorid.

Erstmals veröffentlicht wurde diese Synthese 1953 von Newman und Addor (JACS, 1953, S. 1263; JACS 1955, S. 3789).

Ethylenglykolcarbonat (GC) wurde mittels ultraviolettem Licht bei 60-70°C in Substanz photochloriert und das entstandene CGC durch Vakuumdestillation gereinigt.

VC erhielten Newman und Addor durch Eliminierung mittel Triethylamin in siedendem Ether, wobei das Gemisch über Nacht erhitzt wurde.

Die Isolierung erfolgte durch Abfiltrieren des Triethylammoniumchlorides und anschließende Destillation, die mit 59 %iger Ausbeute ein rohes VC lieferte, welches durch weitere Destillation gereinigt werden musste.

JP 2000/026449 beschreibt die Eliminierung in hochsiedenden Lösungsmitteln (Sdp. 170-300°C). Explizit wird mit Triethylamin in Dibutylcarbonat 20 Stunden bei 50°C umgesetzt. Nach dem Abfiltrieren des Ammoniumchlorides und dem Abdestillieren von überschüssigem Triethylamin wird rohes VC durch einfache Destillation isoliert. Um Spuren vom Aminen zu entfernen wird das VC über eine Silicagelsäule gegeben. Abschließend wird eine Feindestillation durchgeführt. Der Chlorgehalt des so gewonnenen VC wird mit 29 ppm angegeben, während Vergleichsproben > 3000 ppm enthalten. Die Ausbeute beträgt 56 %.

DE-A 19 955 944 beansprucht die Eliminierung in GC als Lösungsmittel (Sdp. 243-244°C). CGC wird in GC vorgelegt und in 1,5 Stunden durch Zugabe von Triethylamin bei 60°C umgesetzt. Nach Abdestillieren von überschüssigem Triethylamin bei 40°C und Verdampfen über einen Dünnschichter bei 100°C wird mit 73 %iger Ausbeute ein farbloses Gemisch aus VC und GC gewonnen. Über die Reinheit werden keine Angaben gemacht.

Die Umsetzungen von CGC in flüssiger Phase liefern nach Abfiltrieren der Salze und einfacher destillativer Trennung vom Lösungsmittel und anderen Verunreinigungen ein rohes Vinylencarbonat, dass mit Resten Chloracetaldehyd, Chlorglykolcarbonat, Dichlorglykolcarbonat und weiteren zum Teil chlorhaltigen organischen Verbindungen verunreinigt ist.

Johnson und Patton beschrieben im JOC, 1960, S. 1042 die Umsetzung von CGC an festen Schüttungen von CaSO₄-Katalysatoren in der Gasphase bei 250°C und 6.67-8kPa (50-60 Torr).

Die Katalysatoren deaktivieren sehr rasch und erreichen bestenfalls einem Umsatz von 35-40 %, bei einer Selektivität von 40-45 %. Höhere oder niedrigere Temperaturen führen zu weniger Umsatz. Die Katalysatoren können durch Abbrennen regeneriert werden.

Granulierte Aktivkohle und granuliertes aktiviertes Aluminiumoxid liefern nur gasförmige Produkte.

DE-A 1 135 452 beschreibt die HCl-Abspaltung von CGC bei 300-400°C. Das CGC wird gasförmig über ein inertes Trägermaterials geleitet, welches mit Elementen der I, II. oder VIII. Nebengruppe des Periodensystem bzw. deren Salzen oder Oxyden belegt ist. Vorzugsweise werden die Chloride des Eisens, Kobalts, Kupfers, besonders bevorzugt Cadmiumchlorid eingesetzt. Geeignete Trägermaterialien sind Bimse und Silicate mit Körnungen von 4 bis 8 mm.

Betrieben werden die Katalysatoren als stationäre Schüttung bei Normaldruck oder Unterdruck und einer Temperatur von 270 bis 450, bevorzugt von 300-400°C.

Das Verhalten von CdCl₂ auf Bimsstein wird explizit beschrieben. Der Katalysator ist mit ca. 270 Stunden deutlich standfester und mit 74 % selektiver als die CaSO₄-Kontakte.

Die Belastung betrug 0,15 kg CGC pro L Katalysator und Stunde und der Inertgasstrom lag zwischen 27 bis 671 pro kg CGC. Der Durchschnittliche Umsatz betrug 87 %.

Der Katalysator kann bei 500 bis 700°C mit Luft abgebrannt werden.

Das Gasphasenverfahren zur Herstellung von Vinylencarbonat, liefert nach einer einfachen Destillation ein rohes Vinylencarbonat, dass bezüglich Verunreinigungen den Flüssigverfahren sehr ähnlich ist.

In Bezug auf den destillativen Reinigungsaufwand sind die Angaben in der Literatur ungenau, so dass man den Aufwand der im Einzelfall getrieben wurde und die Ausbeuteverluste durch die Reinigung nicht abschätzen kann.

Eine hohe Reinheit des VC besonders für die Anwendungen Polymerisation und Additiv für Batterieelektrolyte ist von hoher technischer Bedeutung.

In der US 2 873 230 wird beschrieben, dass VC nach der Methode von Newman und Addor hergestellt auch mit einer 80 bödigen Kolonne nicht ausreichend gereinigt werden kann, um mit Vinylacetat copolymerisiert zu werden und in der Homopolymerisation ungenügende Molekulargewichte erreicht werden.

Chlorhaltige Verunreinigungen werden dafür verantwortlich gemacht. Gegenstand der Anmeldung ist eine Reinigungsmethode die darin besteht, das feindestillierte VC zu verdampfen und gasförmig einer thermischen Behandlung bei 200 bis 450°C zuzuführen. Das so gewonnene VC wird nochmals feindestilliert, erst dann erreicht man eine Reinheit für befriedigende Polymerisationsergebnisse.

Huang et. al. beschreiben im Chin. J. Polm. Sci. (1990) 8 (3), 197-203, dass man VC hergestellt nach der Methode von Newman und Addor, nach dessen Isolierung durch Filtration und Abdestillieren des Lösungsmittels 1 Stunde mit ca. 4 % NaBH4 bei 64°C rührt und dann erst einer Feindestillation unterzieht. Diese Prozedur muss wiederholt werden, um verfärbungsstabiles gut polymerisierbares Material zu gewinnen.

Beide Literaturstellen gehen nicht genau auf den Gehalt an Verunreinigungen ein, der im reinen VC verblieben ist. Verluste durch die Isolierungsprozedur sind ebenfalls offen.

GB-A 899 205 beschreibt, die Reinigung von nach Newman und Addor hergestelltem VC durch mehrmalige Schmelzekristallisation. Um Polymere mit hohem Molekulargewicht zu bekommen muss man VC mit einem Schmelzpunkt größer 21°C einsetzten, dass man durch vierfache Kristallisation erhält. Auch hier wird nicht direkt auf die Reinheit des VC eingegangen, ebenso wenig auf den Verbleib der Mutterlaugen. Da das VC wie in JACS 75, 1263 (1953) beschrieben hergestellt worden ist, wurde destilliertes VC in der Kristallisation verwendet.

Zief und Ruch beschreiben im Journal of Chemical Education (1963, Vol. 40, S. 351-2) die Reinigung von VC durch Zonenschmelzen. Ein Monomer mit einem Schmelzpunkt von 22°C und einem Chlorgehalt von 1-1,8 % erreicht nach einmaligem Zonenschmelzen einen Chlorgehalt von 500 ppm, nach 3 weiteren Durchläufen durch die Zonenschmelzapparatur erreichte das VC einen Chlorgehalt von 50 ppm. Je verunreinigter das VC desto mehr Zonenschmelzläufe benötigt das Material, daher erscheint den Autoren eine vorgelagerte Destillation als sinnvoll.

JP 2002-322171 beschreibt die Kombination von Destillation und Kristallisation zur Reinigung von VC. Zur Kristallisation werden Lösungsmittelgemische aus einer aromatischen Komponente und einem aliphatischen Kohlenwasserstoff beansprucht. Die Ausbeute über Destillation und Kristallisation in den Beispielen ist 60 und 83 %. Die Reinheit liegt oberhalb 99,95 %. Verunreinigungen an Ethylenglykolcarbonat von 400 und 25 ppm und Chloridgehalte von 15 ppm verblieben im VC.

JP 2002-346303 beschreibt die Kristallisation von VC aus Lösungen unter Minimierung der an der Gefäßwand anhaftenden Kristalle. Als Lösemittel werden Gemische aus Toluol und Hexan eingesetzt. Es werden Reinheiten von 99,8 % bis zu 99,93 % bei Ausbeuten von 14 % bis zu 81 % beschrieben.

JP 2002-226475 beschreibt die Kristallisation von VC aus Lösungsmittelgemischen, die aus einem polaren Lösungsmittel und/oder einer aromatischen Komponente und einem aliphatischen Kohlenwasserstoff gebildet werden. In den Beispielen findet man wieder Toluol-Hexan-Gemische. Mit 93,2 %iger Ausbeute wird ein 99,94 %iges VC erhalten, das 11 ppm Chlorid enthält.

In den Anmeldungen zur Reinigung durch Kristallisation, wird entweder mit größeren Mengen an Lösungsmitteln gearbeitet, oder der Kristallisationsvorgang muss mehrmals wiederholt werden um hohe Reinheiten zu erzielen.

Wenn mit Lösungsmittel gearbeitet wird, so muss das VC abschließend nochmals destilliert werden, will man beträchtliche Reste Lösungsmittel vom VC entfernen.

Anzumerken ist, dass die Analysenmethoden zur Reinheitsbestimmung in der Literatur nicht näher beschrieben werden, so dass die Reinheitsangaben nicht eindeutig sind.

Unstabilisiertes reines VC neigt zur Polymerisation, daher wird es mit Stabilisatoren wie BHT(= Butylhydroxytoluol) in den Handel gebracht. Je reiner das Monomer, umso empfindlicher ist es gegenüber ungewollter Polymerenbildung.

Aufgabe der Erfindung ist die Bereitstellung von hochreinen Vinylencarbonat bzw. die Entwicklung eines Verfahrens zur Reinigung von Vinylencarbonat.

Überraschenderweise wurde gefunden, dass ein hoher Reinheitsgrad von VC erreicht wird, wenn vor die Feindestillation eine einfache thermische Behandlung mit Harnstoff durchgeführt wird. Das gelingt, unabhängig davon, ob das technische VC durch Eliminierung in der flüssigen Phase oder in der Gasphase gewonnen wurde. Führt man dieses VC einer Schmelzekristallisation zu, so erhält man VC höchster Reinheit, die dabei anfallende Mutterlauge wird in die vorgeschalteten Reinigungsschritte recycliert, so dass nur minimale Verluste an VC auftreten.

Gegenstand der Erfindung ist daher ein Verfahren zur Reinigung von Vinylencarbonat, bei dem das aufzureinigende V inylencarbonat
a) bei einer Temperatur im Bereich von 25 bis 180°C mit 0,1 bis 30 Gew.% Harnstoff, bezogen auf Vinylencarbonat, in Kontakt gebracht wird,
b) eventuell ausgefallener Feststoff abfiltriert wird,
c) das so gereinigte Vinylencarbonat über eine Kolonne destilliert wird und
d) aus dem Destillat durch Schmelzkristallisation das aufgereinigte Vinylcarbonat anfällt.

Die thermische Behandlung im Schritt a) erfolgt meist unter Rühren bei Temperaturen zwischen 25 und 180°C, bevorzugt zwischen 60 und 160°C, besonders bevorzugt zwischen 90 und 140°C.

Relativ zum Vinylencarbonat werden 0,1-30 Gew.%, bevorzugt 1-10 Gew.-%, besonders bevorzugt 2-6 Gew.-% Harnstoff zugesetzt.

Der Zusatz von Harnstoff kann in Anwesenheit oder ohne Zusatz von Lösungsmitteln erfolgen. Als Lösungsmittel sind beispielsweise zu nennen, Dimethylacetamid, N-Methylpyrolidon, Dimethylformamid und DMSO.

Nach der thermischen Behandlung wird das Vinylencarbonat vom Rückstand in Schritt c) abdestilliert. Dies kann als Batch-Destillation aus einem Behälter über eine Kolonne mit mindestens 10, bevorzugt mindestens 20, besonders bevorzugt mindestens 30 Böden geschehen.

Als Kolonneneinbauten sind alle dem Fachmann bekannten Möglichkeiten geeignet, z.B. Glockenböden, Siebböden, ferner Füllkörperschüttung wie z.B., Raschigringe, Pallringe, Berlsättel, außerdem Kreuzkanalstruckturen wie z.B. die Packungen der Firmen Sulzer und Montz.

Die durch das erfindungsgemäße Verfahren anfallenden leichtsiederreichen Vorläufe der Feindestillation werden vorteilhafter Weise gesammelt und zur verlustarmen Ausschleusung jener Leichtsieder getrennt destilliert, wobei das so wieder gewonnene VC zur thermischen Behandlung in Schritt a) und/oder Feindestillation in Schritt b) recycliert wird.

Das so gewonnene VC wird im erfindungsgemäßen Verfahren in Schritt d) mit einem Gehalt zwischen 99,0 und 99,99, bevorzugt zwischen 99,5 und 99,9 % einer Schmelzkristallisation unterworfen.

Die Schmelzekristallisation kann in technischen Anlagen durchgeführt werden, wie sie dem Fachmann bekannt sind, zu nennen sind im besonderen Rohrbündelkristaller.

Das VC fällt nach dem Isolieren in sehr hoher Reinheit, mit Restchloridgehalten < 10 ppm an.

Die bei der Kristallisation anfallende Mutterlauge kann, bevorzugt inklusive der Anschmelzflüssigkeit, direkt in die Destillation in Schritt c) und/oder thermische Behandlung in Schritt a) recycliert wird.

Überraschenderweise wurde gefunden, dass unstabilisiertes VC im festen Zustand über lange Zeit unverändert lagerbar ist.

Im Folgenden wird das erfindungsgemäße Verfahren anhand einiger Beispiele illustriert, wobei die Beispiele jedoch nicht als Einschränkung des Erfindungsgedankens zu verstehen sind.

### Beispiele:

### Eingesetzte Apparatur:

Die Destillationsapparatur bestand aus einem ölbeheizten 15L-Planschlifftopf mit Ankerrührer, Kolonne, Rückflussteiler, Kondensator und einer Vorrichtung zum Einstellen eines konstanten Vakuums. Vor der Vakuumpumpe befand sich eine auf -78°C gekühlte Kühlfalle. Planschlifftopf, Kolonne, Rückflussteiler und Kondensator waren aus Glas, der Ankerrührer aus Teflon.

Die Kolonne hatte eine 1500 mm lange Sulzer DX Packung aus Hastelloy C mit einem Durchmesser von 50 mm. Packungen dieses Typs haben Trennleistungen zwischen 15 und 30 Böden pro Meter.

Die Apparatur wurde vor und nach dem Beschicken und vor dem Betrieb immer mit Stickstoff inertisiert.

### Beispiel 1 Reinigung von VC:

Als Ausgangsstoff wurde durch eine Vordestillation ohne Kolonne im Wesentlichen lediglich von polymeren Verunreinigungen befreites rohes VC eingesetzt.

Dieses rohe VC war ca. 97 %ig und hatte einen Gehalt an organischem und anorganischem Chlor von ca. 0,5 % bis 1 %.

Die gaschromatographische Analyse erfolgte mittel eines HP 6890. Getrennt wurde über eine 50 Meter lange CP-Sil 8 CB mit einem ID von 0,53 mm und einer FD von 1,0 µm.

Trägergas war Stickstoff mit einem Vordruck von 3,5 10° Pa (5 psi). Der Injektor wurde mit einem Flow von 138 ml/min und einem Split von 30/1 betrieben. Injiziert wurde 1 µL VC pur.

Die Injektortemperatur betrug 220°C, die Detektortemperatur 320°C. Das Temperaturprogramm startete mit 50°C, heizte mit 5°C/min bis 250°C auf.

Ausgewertet wurde nach der Norm %-Methode.

### 1. Verfahrensstufe: Behandlung mit Harnstoff bei 140°C, abgesaugt, mit NMP verdünnt:

12060 g rohes VC wurden mit 200 g Harnstoff versetzt und unter Stickstoff 2 Stunden bei 140°C gerührt. Nach Abkühlen auf ca. 30-40°C wurden 235 g Feststoff abfiltriert, 11743 g Flüssigkeit in die oben beschriebene Destillationsapparatur überführt und mit 1000 g NMP versetzt.

Das Gemisch wurde bei ca. 35 mbar Druck zum Rückfluss erhitzt und anschließend mit einem Rücklaufverhältnis von 30/1 Vorlauf abdestilliert.

Innerhalb von 2,5 Stunden fielen so ca. 160 g Destillat an, dass laut GC-Analyse zu 96 % aus VC bestand. In den folgende 3,5 Stunden fielen ca. 400 g eines Destillates an, dass zu 97,5 % aus VC bestand, gefolgt von ca. 470 g Destillat mit 99,4 % VC Gehalt, die in 2,5 Stunden überdestillierten.

Nun wurde mit einem Rücklaufverhältnis von 5/1 der Hauptlauf abgenommen. In 26 Stunden destillierten ca. 9600 g eines 99,9 %igen VC über, dass einen Chlorgehalt unterhalb 50 ppm hatte.

Es blieben ca. 1100 g Sumpf zurück, mit einem VC-Gehalt von weniger als 0,5 %.

Die Kühlfalle war praktisch leer geblieben.

Die Massenbilanz war praktisch quantitativ, VC wurde zu 93 % wieder gefunden.

84 % des Vinylencarbonates sind in der Hauptfraktion angefallen.

### 2. Verfahrensstufe Kristallisation im statischen Kristaller:

Der Kristallisator bestand aus einem 400 mm langen thermostatisierten Glasrohr mit einem Innendurchmesser von 30 mm. Am unteren Ende war eine Lochscheibe zur Fixierung der Kristalle und ein Ablasshahn für die Mutterlauge angebracht. Unter dem Absperrhahn befand sich eine argongespülte Wechselvorlage. Am oberen Ende konnte die Gasphase durch Spülen mit Argon ersetzt werden, ferner reichte ein kühlbarer Kunststofffinger in den Innenraum, mittels dessen Hilfe die Kristallisation gezielt initiiert werden konnte.

302 g des Destillates aus der Verfahrensstufe 1 wurden in den Kristaller gefüllt und die Gasphase durch Spülen mit Argon verdrängt. Das Vinylencarbonat wurde mit einem 19°C kalten Ölkreislauf herunter gekühlt. Dann wurde durch Abkühlen des Kühlfingers die Kristallisation in Gang gesetzt und 4 Stunden laufen gelassen.

Man beobachtet ein rasches Ausbreiten einer Kristallfront vom Kühlfinger über die gesamte Wärmetauscherfläche. Dann wuchsen die Kristalle auffallend kompakt nach innen.

Nach 4 Stunden wird durch öffnen des Hahn am unteren Ende zunächst die Flüssigkeit unter der Lochblende im ungekühlten Teil des Rohes, dann die Mutterlauge abgelassen und getrennt aufgefangen.. Anschließend wurde die Vorlage erneut gewechselt und bei geöffnetem Hahn in 1 h mit einer linearen Rampe der Ölkreislauf auf 22°C aufgewärmt und eine weiter Stunde bei 22°C gehalten, wobei die Kristalle durch anschwitzen gereinigt werden. Zum Schluss wurde die Hauptfraktion bei 30°C in eine weiter Vorlage abgeschmolzen.

Das Edukt war ca. 99,9%ig, hatte einen Chlorgehalt < 50 ppm und einen Wassergehalt von ca. 100 ppm.

24 g Vorlauf wurden aufgefangen, mit einem Gehalt an VC von 99,87 %, einem Chlorgehalt von ca. 110 ppm und einem Wassergehalt von ca. 230 ppm.

Es fielen 56 g Mutterlauge an, mit einem Gehalt an VC von 99,8 %, einem Chlorgehalt von 160 ppm und einem Wassergehalt von 330 ppm.

Die Schwitze wog 35 g, hatte einen Gehalt von 99,9 % an VC, 70 ppm Chlor und 110 ppm Wasser.

Die Produktschmelze wog 187 g, hatte einen Gehalt von 99,99 % an VC, lag mit 3 ppm Chlor an der Nachweisgrenze und enthielt 10 ppm Wasser.

Mutterlauge, Vorlauf und Schwitze können direkt in die Harnstoffbehandlung oder die Destillation recycliert werden und sind somit kein Verlust.

### Beispiel 2 Vergleich der Eigenschaften von flüssigen und festen VC:

Proben von Vinylencarbonat gereinigt nach Beispiel 1 wurden unstabilisiert unter Ausschluss von Licht als Flüssigkeit bei 20°C und in kristalliner Form bei 5°C gelagert.

### Flüssiges VC:

Die unstabilisierten Proben Vinylencarbonat zeigen nach 70 Tagen schwache bis starke Gelbfärbungen und schwache Trübungserscheinungen. Der Gehalt, gemessen mit ISTD fiel auf bis zu 96 %. Unstabilisiert ist hochreines Vinylencarbonat im flüssigen Zustand nicht lagerstabil.

### Festes VC:

Die bei 5°C im festen Zustand gelagerten Proben waren alle auch nach 365 Tagen nach dem aufschmelzen farblos und klar. Die Analysen zeigten keine Veränderungen der Qualität. Unstabilisiertes Vinylencarbonat ist im festen Zustand lagerstabil!

## Patentansprüche

1. Verfahren zur Reinigung von Vinylencarbonat, bei dem das aufzureinigende Vinylencarbonat
a) bei einer Temperatur im Bereich von 25 bis 180°C mit 0,1 bis 30 Gew.% Harnstoff, bezogen auf Vinylencarbonat, in Kontakt gebracht wird,
b) eventuell ausgefallener Feststoff abfiltriert wird,
c) das so gereinigte Vinylencarbonat über eine Kolonne destilliert wird und
d) aus dem Destillat durch Schmelzkristallisation das aufgereinigte Vinylcarbonat anfällt.

2. Verfahren nach Anspruch 1, **dadurch gekennzeichnet, dass** der Harnstoff zu 1 bis 10 Gew.-%, bezogen auf Vinylencarbonat, dem Vinylencarbonat zugesetzt wird.

## Claims

1. Process for the purification of vinylene carbonate, in which the vinylene carbonate to be purified
a) at a temperature in the range of 25 to 180°C is contacted with 0.1 to 30% by weight of urea, based on vinylene carbonate,
b) any precipitated solid is filtered off,
c) the vinylene carbonate purified in this way is distilled via a column and
d) the purified vinyl carbonate is obtained from the distillate by melt crystallization.

2. Process according to Claim 1, **characterized in that** the urea is added in an amount of 1 to 10% by weight, based on vinylene carbonate, to the vinylene carbonate.

## Revendications

1. Procédé de purification de carbonate de vinylène, selon lequel le carbonate de vinylène à purifier
a) est mis en contact à une température dans la plage allant de 25 à 180 °C avec 0,1 à 30 % en poids d'urée, par rapport au carbonate de vinylène,
b) le solide éventuellement précipité est éliminé par filtration,
c) le carbonate de vinylène ainsi purifié est distillé par une colonne et
d) le carbonate de vinyle purifié est produit à partir du distillat par cristallisation à l'état fondu.

2. Procédé selon la revendication 1, **caractérisé en ce que** l'urée est ajoutée au carbonate de vinylène à hauteur de 1 à 10 % en poids, par rapport au carbonate de vinylène.
